# EUROPÄISCHE PATENTSCHRIFT

(11) **EP 0 786 244 B1**
(45) Veröffentlichungstag und Bekanntmachung des Hinweises auf die Patenterteilung: **19.09.2001**
(21) Anmeldenummer: 96116146.0
(22) Anmeldetag: 09.10.1996
(51) Int. Cl.: A61K 7/13, C07D 231/38

(54) **Oxidationshaarfärbemittel mit einem Gehalt an 3, 4, 5-Triaminopyrazolderivaten sowie neue 3,4,5-Triaminopyrazolderivate**
Oxidation hair-dye comprising 3,4,5 triaminopyrazole derivatives, and novel 3,4,5 triaminopyrazole derivatives
Colorant d'oxydation pour cheveux, comprenant des dérivés de 3,4,5 triaminopyrazoles, ainsi que les dérivés de 3,4,5 triaminopyrazoles

(30) Priorität: 25.11.1995 DE 19543988
(43) Veröffentlichungstag der Anmeldung: 30.07.1997
(73) Patentinhaber: Wella Aktiengesellschaft, 64274 Darmstadt (DE)
(72) Erfinder: Löwe, Isolde, 64625 Bensheim (DE); Gerstung, Stefan, Dr., 36142 Tann (DE); Balzer, Wolfgang R., Dr., 64665 Alsbach (DE)

(56) Entgegenhaltungen:
- EP-A- 0 375 977
- DE-A- 4 234 885
- DE-A- 4 234 886
- J. PRAKT. CHIMIE, Bd. 323, Nr. 2, 1981, LEIPZIG, Seiten 332-336, XP000645676 SCHÄFER ET AL.: "SUBSTITUIERTE 4-NITROPYRAZOLE AUS NITROKETENAMINALEN"

## Beschreibung

Gegenstand der Erfindung sind Mittel zur oxidativen Färbung von Haaren auf der Basis von 3,4,5-Triaminopyrazolderivaten als Entwicklersubstanz sowie neue 3,4,5-Triaminopyrazolderivate.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

EP-A-0 375 977 offenbart ein Mittel zur oxidativen Färbung von Haaren auf der Basis einer Entwicklersubstanz-Kupplersubstanz-Kombination, welche als Entwicklersubstanz ein Diaminopyrazolderivat darstellt, unter der Voraussetzung, daß die Aminogruppen in 3,4-oder 4,5-Stellung stehen, sowie neue Diaminopyrazolderivate.

Als Entwicklersubstanzen werden insbesondere 2,5-Diaminotoluol, 2,5-Diaminophenylethylalkohol, p-Aminophenol und 1,4-Diaminobenzol eingesetzt. Von den vorzugsweise verwendeten Kupplersubstanzen sind Resorcin, 4-Chlorresorcin, 1-Naphthol, 3-Aminophenol, 5-Amino-2-methylphenol und Derivate des m-Phenylen-diamins zu nennen.

An Oxidationsfarbstoffe, die zur Färbung menschlicher Haare verwendet werden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzieltn Haarfärbungen eine gute Licht-, Dauerwell-, Säure und Reibeechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwickler- und Kupplerkomponenten eine breite Palette verschiedener Farbnuancen erzeugt werden kann. Zur Erzielung natürlicher und besonders modischer Nuancen im Rotbereich wird vor allem 4-Aminophenol, allein oder im Gemisch mit anderen Entwicklersubstanzen in Kombination mit geeigneten Kupplungssubstanzen eingesetzt.

Gegen den für den Rotbereich der Farbskala bisher hauptsächlich eingesetzten Entwickler 4-Aminophenol wurden in letzter Zeit Bedenken in bezug auf die physiologische Verträglichkeit erhoben, während die in neuerer Zeit empfohlenen Entwicklersubstanzen, wie zum Beispiel Pyrimidinderivate, in färberischer Hinsicht nicht völlig zufriedenstellen können.

Die in der DE-OS 21 60 317 beschriebenen Pyrazol-derivate, wie zum Beispiel das 3-Amino-1-phenyl-2-pyrazolon-5, färben Haare nur in sehr geringen, für die Haarfärbepraxis unbrauchbaren, Farbtiefen an.

Es bestand daher die Aufgabe, ein Oxidationshaarfärbemittel auf der Basis einer Entwicklersubstanz/Kupplersubstanz-Kombination zur Verfügung zu stellen, in dem eine Entwicklersubstanz für den Rotbereich enthalten ist, welche physiologisch sehr gut verträglich ist und mit üblichen Kupplersubstanzen das Haar in brillanten roten Farbtönen mit einer hohen Farbtiefe färbt.

Hierzu wurde nun gefunden, daß durch ein Mittel zur oxidativen Färbung von Haaren auf der Basis einer Entwicklersubstanz-Kupplersubstanz-Kombination, welche als Entwicklersubstanz ein 3,4,5-Triaminopyrazol der allgemeinen Formel (I), 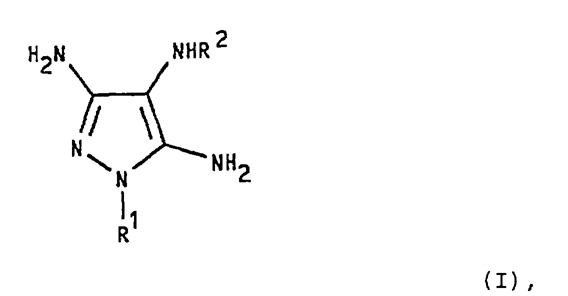 in der R¹ gleich Wasserstoff, Alkyl mit 1 bis 4 Kohlenstoffatomen oder Hydroxyalkyl mit 2 bis 4 Kohlenstoffatomen darstellt und R² Wasserstoff, Alkyl mit 1 bis 4 Kohlenstoffatomen oder Hydroxyalkyl mit 2 bis 4 Kohlenstoffatomen bedeutet, oder dessen physiologisch verträgliche, wasserlösliche Salze enthält, diese Aufgabe in hervorragender Weise gelöst wird,

In dem Haarfärbemittel sollen die Entwicklersubstanzen der Formel (I), von denen das 1-Methyl-3,4,5-triaminopyrazol, das 3,5-Diamino-1-methyl-4-methylaminopyrazol und das 3,5-Diamino-4-(2'-hydroxyethyl)amino-1-methyl-pyrazol bevorzugt ist, in einer Menge von 0,01 bis 3,0 Gewichtsprozent, vorzugsweise in einer Menge von 0,1 bis 2,5 Gewichtsprozent, enthalten sein.

Obwohl die vorteilhaften Eigenschaften der hier beschriebenen neuen Entwicklersubstanzen es nahelegen, diese als alleinige Entwicklersubstanz zu verwenden, ist es selbstverständlich auch möglich, die Entwicklersubstanzen der Formel (I) gemeinsam mit bekannten Entwicklersubstanzen, wie zum Beispiel 1,4-Diaminobenzol, 2,5-Diaminotoluol oder 2,5-Diaminophenylethylalkohol, einzusetzen.

Als Kupplersubstanen kommen vorzugsweise α-Naphthol, Resorcin, 4-Chlorresorcin, 2-Methylresorcin, 2-Amino-4-(2'-hydroxyethyl) amino-anisol, 2,4-Diaminophenylalkohol, m-Phenylendiamin, 5-Amino-2-methylphenol, 2,4-Diaminophenoxyethanol, 4-Amino-2-hydroxyphenoxyethanol, 3-Aminophenol, 3-Amino-2-methylphenol, 4-Hydroxy-1,2-methylendioxybenzol, 4-Amino-1,2-methylendioxybenzol, 4-(2'-Hydroxyethyl)amino-1-2,-methylendioxybenzol, 2,4-Diamino-5-fluortoluol, 4-Amino-5-fluor-2-hydroxytoluol, 2,4-Diaminobenzylalkohol, 2,4-Diamino-phenetol, 2,4-Diamino-5-methylphenetol, 4-Hydroxyindol, 3-Amino-5-hydroxy-2,6-dimethoxypyridin und 3,5-Diamino-2,6-dimethoxypyridin in Betracht.

Die Kuppler- und Entwicklersubstanzen können in dem Haarfärbemittel jeweils einzeln oder im Gemisch miteinander enthalten sein.

Die Gesamtmenge der in dem hier beschriebenen Haarfärbemittel enthaltenen Entwicklersubstanz-Kupplersubstanz-Kombination beträgt 0,1 bis 5,0 Gewichtsprozent, wobei eine Menge von 0,5 bis 4,0 Gewichtsprozent bevorzugt ist. Die Entwicklerkomponenten werden im allgemeinen in etwa äquimolaren Mengen, bezogen auf die Kupplerkomponenten, eingesetzt. Es ist jedoch nicht nachteilig, wenn die Entwicklerkomponenten diesbezüglich in einem gewissen Überschuß oder Unterschuß vorhanden sind.

Weiterhin kann das erfindungsgemäße Haarfärbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'aminophenyl)-(4'-imino-2",5"-cyclohexadien-1"-yliden)-methyl]-2-methylaminobenzol-monohydrochlorid (C.I.42 510) und 4-[(4'amino-3'-methyl-phenyl)-(4"-imino-3"-methyl-2",5"-cyclohexadien-1"-yliden)-methyl]-2-methyl-aminobenzolmohohydrochlorid (C.I. 42 520), aromatische Nitrofarbstoffe wie 2-Nitro-1,4-diaminobenzol, 2-Amino-4-nitrophenol, 2-Amino-5-nitrophenol, 2-Amino-4-6-dinitro-phenol, 2-Amino-5-(2'-hydroxyethyl)amino-nitrobenzol und 2-Methylamino-5-bis-(2'-hydroxyethyl)amino-nitrobenzol, Azofarbstoffe wie 6-[(4'-Aminophenyl) azo]-5-hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoanthrachinon enthalten. Die Haarfärbemittel können diese Farbkomponenten in einer Menge von etwa 0,1 bis 4,0 Gewichtsprozent enthalten.

Selbstverständlich können die Kuppler- und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem Haarfärbemittel noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des neuen Haarfärbemittels kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkohlsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Petrolatum, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert von 6,0 bis 11,5 auf, wobei die basische Einstellung mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen zum Beispiel Phosphorsäure sowie Essigsäure oder andere organische Säuren, wie zum Beispiel Zitronensäure oder Weinsäure, in Betracht.

Für die Anwendung zur oxidativen Färbung von Haaren vermischt man das vorstehend beschriebene Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form einer 3- bis 12-prozentigen, vorzugsweise 6-prozentigen, wäßrigen Lösung, aber auch Luftsauerstoff in Betracht. Wird eine 6-prozentige Wasserstoffperoxid-Lösung als Oxidationsmittel verwendet, so beträgt das Gewichtsverhältnis zwischen Haarfärbemittel und Oxidationsmitte 5:1 bis 1:2, vorzugsweise jedoch 1:1. Größere Mengen an Oxidationsmittel werden vor allem bei höheren Farbstoffkonzentrationen im Haarfärbemittel oder wenn gleichzeitig eine stärkere Bleichung des Haares beabsichtigt ist verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die in dem erfindungsgemäßen Haarfärbemitteln enthaltenen 3,4,5-Triamino-pyrazole der allgemeinen Formel (I) lassen sich gut nach folgendem Schema herstellen:

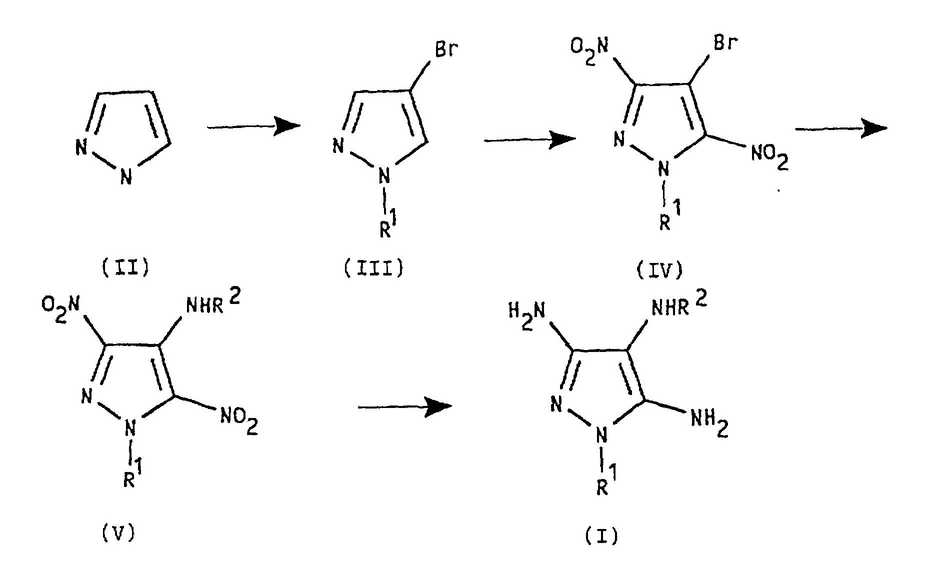

Die Salze der Verbindungen der Formel (I) sollen in dem Haarfärbemittel entweder als freie Basen oder in Form ihrer physiologisch verträglichen Salze mit anorganischen oder organischen Säuren, wie zum Beispiel Salzsäure, Milchsäure oder Zitronensäure, eingesetzt werden. Die Verbindungen der Formen (I) sind gut in Wasser löslich und sie weisen zusätzlich eine ausgezeichnete Lagerstabilität, insbesondere als Bestandteil der hier beschriebenen Haarfärbemittel, auf.

Das erfindungsgemäße Haarfärbemittel mit einem Gehalt an 3,4,5-Triaminopyrazolderivaten als Entwicklersubstanzen ermöglicht Haarfärbungen mit ausgezeichneter Farbechtheit, insbesondere was die Licht-, Wasch- und Reibeechtheit anbetrifft.

Hinsichtlich der färberischen Eigenschaften bieten die erfindungsgemäßen Haarfärbemittel Möglichkeiten, die weit über einen Ersatz der üblicherweise verwendeten 4-Aminophenole hinausgehen. So lassen sich brillante Rottöne mit außerordentlicher Farbtiefe erzeugen, wie sie mit den gängigen Farbkomponenten nicht zu erzielen sind. Neben dieser Anwendung im hochmodischen Bereich können aber auch durch die Verwendung in Kombination mit geeigneten Kupplungskomponenten natürliche Farbtöne erzeugt werden, ohne daß eine weitere Entwicklungskomponente vom Typ der p-Phenylendiamine erforderlich wäre.

Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Gegenstand der vorliegenden Patentanmeldung sind ferner die neuen 3,4,5-Triaminpyrazolderivate der allgemeinen Formel (I), wobei insbesondere 1-Methyl-3,4-5-triaminopyrazol, 3,5-Diamino-1-methyl-4-methylamino-pyrazol und 3,5-Diamono-4-(2'-hydroxyethyl) amino-1-methylpyrazol zu nennen sind.

Die nachfolgenden Beispiele sollen den Gegenstand der Erfindung näher erläutern, ohne ihn darauf zu beschränken.

### BEISPIELE

### Herstellungsbeispiele

### Beispiel 1: Synthese von 4-Amino-3,5-dinitro-1-methylpyrazolen der Formel (V)

### Allgemeine Vorschrift

5 g (20 mmol) 4-Brom-3,5-dinitro-1-methylpyrazol werden in 20 ml Dimethylsulfoxid (DMSO) mit dem dreifachen molaren Überschuß an Alkylamin versetzt und 2 Stunden auf dem Wasserbad erwärmt. Anschließend wird das Reaktionsprodukt auf Eis gegossen und der Niederschlag abfiltriert und aus Ethanol umkristallisiert.
**1a.) 4-Benzylamino-3,5-dinitro -1-methylpyrazol**
   Verwendetes Amin: Benzylamin
   Es werden 3,46 g (= 63 Prozent der Theorie)4-Benzylamino-3,5-dinitro-1-methylpyrazol in Form gelber Kristalle erhalten.
   Schmelzpunkt: 92°C
**1b.) 4-Methylamino-3,5-dinitro-1-methylpyrazol**
   Verwendetes Amin: Methylamin
   Es werden 2,71 g(= 67 Prozent der Theorie) 4-Methylamino-3,5-dinitro-1-methylpyrazol in Form gelber Kristalle erhalten.
   Schmelzpunkt: 128°C.
**1c.) 4- (2'-Hydroxyethyl) amino-3,5-dinitro-1-methylpyrazol**
   Verwendetes Amin: Ethanolamin
   Es werden 2,22 g (= 50 Prozent der Theorie) 4-(2'-Hydroxyethyl)amino-3,5-dinitro-1-methylpyrazol in Form gelber Kristalle erhalten.
   Schmelzpunkt: 101 bis 103°C

### Beispiel 2: Synthese von 1-Methyl-3,4,5-triaminopyrazolen der Formel (I)

### Allgemeine Vorschrift

10 mmol der entsprechenden Nitroverbindung 1a., 1b. oder 1c. werden in Methanol unter Verwendung eines Palladium-Aktivkohle-Katalysators (10 %) bei Raumtemperatur (20 bis 30°C) hydriert. Nach Beendigung der Reaktion wird vom Katalysator in ein Gemisch aus Schwefelsäure und Wasser abfiltriert. Nach Zugabe von Methanol kristallisieren die entsprechenden Sulfate aus.
**2a.) 1-Methyl-3,4,5-Triaminopyrazol-sulfat**
   Verwendete Nitroverbindung: 1a)
   Man erhält 2 g (= 89 Prozent der Theorie) 1-Methyl-3,4,5-Triaminopyrazol-sulfat in Form farbloser Kristalle, welche bei 205°C unter Zersetzung schmelzen.
**2b.) 3,5-Diamino-1-methyl-4-methylaminopyrazol-sulfat**
   Verwendete Nitroverbindung: 1b)
   Man erhält 2 g (= 84 Prozent der Theorie) 3,5-Diamino-1-methyl-4-methylaminopyrazol-sulfat in Form farbloser Kristalle, welche bei 214°C unter Zersetzung schmelzen.
**2c.) 3,5-Diamino-4-(2'-hydroxyethyl)amino-1-methylpyrazol-sulfat**
   Verwendete Nitroverbindung: 1c.)
   Man erhält 1,6 g (= 59 Prozent der Theorie) 3,5-Diamino-4-(2'-hydroxyethyl)amino-1-methylpyrazolsulfat in Form farbloser Kristalle, welche bei 198°C unter Zersetzung schmelzen.

### Haarfärbebeispiele

### Beispiel 3: Haarfärbemittel in Cremeform

| | |
|---|---|
| 1,40 g | 1-Methyl-3,4,5-triaminopyrazol-sulfat |
| 0,67 g | α-Naphthol |
| 0,28 g | 2-Amino-4-(2'-hydroxyethyl)amino-anisolsulfat |
| 15,00 g | Cetylalkohol |
| 3,50 g | Natrium-laurylalkohol-diglycolethersulfat (28prozentige wäßrige Lösung) |
| 0,30 g | Natriumsulfit, wasserfrei |
| 0,19 g | NaOH (10prozentige wäßrige Lösung) |
| 1,77 g | Ammoniak (25prozentige wäßrige Lösung) |
| 76,89 g | Wasser |
| **100,00 g** | |

50 g des vorstehenden Haarfärbemittels werden kurz vor dem Gebrauch mit 50 g Wasserstoffperoxidlösung (6-prozentig) vermischt und das Gemisch wird anschließend auf blonde Naturhaare aufgetragen. Nach einer Einwirkzeit von 30 Minuten bei 40 Grad Celsius wird das Haar mit Wasser gespült und getrocknet. Das Haar hat eine intensive auberginefarbene Färbung erhalten.

### Beispiel 4: Haarfärbemittel in Cremeform

| | |
|---|---|
| 1,44 g | 3,5-Diamino-1-methyl-4-methylaminopyrazolsulfat |
| 0,31 g | 5-'Amino-2-methyl-phenol |
| 0,36 g | α-Naphthol |
| 15,00 g | Cetylalkohol |
| 3,50 g | Natrium-laurylalkohol-diglycolethersulfat (28prozentige Lösung) |
| 0,30 g | Natriumsulfit, wasserfrei |
| 0,19 g | NaOH (10prozentige wäßrige Lösung) |
| 1,00 g | Ammoniak (25prozentige wäßrige Lösung) |
| 77,90 g | Wasser |
| **100,00 g** | |

Man vermischt kurz vor dem Gebrauch 50 g des vorstehend beschriebenen Haarfärbemittels mit 50 g Wasserstoffperoxidlösung (6-prozentig) und läßt die Mischung 30 Minuten bei 40 Grad Celsius auf blonde Naturhaare einwirken. Das Haar wird mit Wasser gespült und getrocknet. Das Haar wird intensiv purpurrot gefärbt.

### Beispiel 5 bis 17: Haarfärbemittel

Es werden Haarfärbelösungen folgender Zusammensetzung hergestellt:

| | |
|---|---|
| 0,025 mol | Entwickler nach Tabelle 1 |
| 0,025 mol | Kuppler nach Tabelle 1 |
| 10,00 g | Laurylalkohol-diglycolethersulfat-Natriumsalz (28prozentige wäßrige Lösung) |
| 10,00 g | Ammoniak (22prozentige wäßrige Lösung) |
| ad 100,00 g | Wasser |
| **100,00 g** | |

Die Haarfärbelösungen werden gemäß Beispiel **3** zubereitet und auf zu 90 Prozent ergraute, menschliche Haare aufgetragen. Die resultierenden Färbungen sind in Tabelle 1 zusammengefaßt.

**Tabelle 1**

| **Bsp.** | **Entwickler der Formel (I) aus Herstellungbsp.** | **Kuppler** | **Farbe** |
|---|---|---|---|
| 5 | 2a) | 5-Amino-2-methyl-phenol | leuchtend rot |
| 6 | 2b) | 5-Amino-2-methyl-phenol | rot |
| 7 | 2c) | 5-Amino-2-methyl-phenol | rot |
| 8 | 2a) | 3-Amino-phenol | orange |
| 9 | 2b) | 3-Amino-phenol | orange |
| 10 | 2c) | 3-Amino-phenol | orange-rosa |
| 11 | 2a) | 2-Amino-4-(2'-hydroxyehtyl)amino-anisol-sulfat | dunkel blaugrau |
| 12 | 2b) | 2-Amino-4-(2'-hydroxyehtyl)amino-anisol-sulfat | blaugrau |
| 13 | 2c) | 2-Amino-4-(2'-hydroxyehtyl)amino-anisolsulfat | grauviolett |
| 14 | 2a) | α-Naphtol | goldgelb |
| 15 | 2b) | α-Naphtol | goldorange |
| 16 | 2a) | 2,4-Diamino-5-fluortoluol | dunkelviolett |
| 17 | 2b) | 2,4-Diamino-5-fluortoluol | violett |

Alle Prozentangaben stellen, soweit nicht anders angegeben, Gewichtsprozente dar.

## Patentansprüche

1. Mittel zur oxidativen Färbung von Haaren auf der Basis einer Entwicklersubstanz-Kupplersubstanz-Kombination, **dadurch gekennzeichnet, daß** es als Entwicklersubstanz ein 3,4,5-Triaminopyrazol der allgemeinen Formel (I) 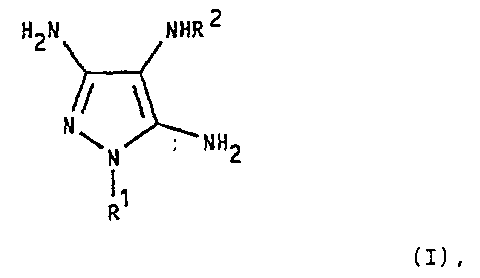 in der R¹ Wasserstoff, Alkyl mit 1 bis 4 Kohlenstoffatomen oder Hydroxyalkyl mit 2 bis 4 Kohlenstoffatomen darstellt und R² Wasserstoff, Alkyl mit 1 bis 4 Kohlenstoffatomen oder Hydroxyalkyl mit 2 bis 4 Kohlenstoffatiomen bedeutet, oder dessen physiologisch verträgliche, wasserlösliche Salze enthält.

2. Mittel nach Anspruch 1, **dadurch gekennzeichnet, daß** die Entwicklersubstanz der allgemeinen Formel (I) ausgewählt ist aus 1-Methyl-3,4,5-triaminopyrazol, 3,5-Diamino-1-methyl-4-methylamino-pyrazol und 3,5-Diamino-4-(2'-Hydroxyethyl)amino-1-methyl-pyrazol.

3. Mittel nach Anspruch 1 oder 2, **dadurch gekennzeichnet, daß** die Entwicklersubstanz der Formel (I) in einer Menge von 0,01 bis 3,0 Gewichtsprozent enthalten ist.

4. Mittel nach einem der Ansprüche 1 bis 3, **dadurch gekennzeichnet, daß** die Kupplersubstanz ausgewählt ist, aus α-Naphthol, Resorcin, 4-Chlorresorcin, 2-Methylresorcin, 2-Amino-4- (2' -hydroxyethyl)aminoanisol, 5-Amino-2-methylphenol, 3-Amino-2-methylphenol, 4-Hydroxy-1,2-methylendioxybenzol, 4-Amino-1,2-methylendioxybenzol, 4-(2'-Hydroxyethyl)amino-1,2-methylendioxybenzol, 2,4-Diamino-phenetol, 2,4-Diamino-5-methylphenetol, 2,4-Diamino-5-fluortoluol, 4-Amino-5-fluor-2-hydroxytoluol, 2,4-Diaminobenzylalkohol, m-Phenylendiamin, 2,4-Diaminophenoxyethanol, 4-Hydroxyindol, 3-Amino-5-hydroxy-2,6-dimethoxypyridin und 3,5-Diamino-2,6-dimethoxy-pyridin.

5. Mittel nach einem der Ansprüche 1 bis 4, **dadurch gekennzeichnet, daß** die Gesamtmenge der Entwicklersubstanz-Kupplersubstanz-Kombination 0,1 bis 5,0 Gewichtsprozent beträgt.

6. Mittel nach einem der Ansprüche 1 bis 5, **dadurch gekennzeichnet, daß** es eine zusätzliche Farbkomponente enthält, die ausgewählt ist aus 6-Amino-2-methylphenol, 2-Amino-5-methylphenol, 4-[(4'-aminophenyl)-(4'-imino-2",5"-cyclohexadien-1"-yliden)-methyl]-2-methyl-aminobenzol-monohydrochlorid (C.I. 42 510), 4-[(4'-amino-3'-methylphenyl)-(4"-imino-3"-methyl-2",5"-cyclohexachen-1"-yliden)-methyl]-2-methyl-aminobenzol-monohydrochlorid (C.I. 42 520), 2-Nitro-1,4-diaminobenzol, 2-Amino-4-nitro-phenol, 2-Amino-5-nitrophenol, 2-Amino-4,6-dinitrophenol, 2-Amino-5-(2'-hydroxyethyl)amino-nitrobenzol, 2-Methylamino-5-bis-(2'-hydroxyethyl)amino-nitrobenzol, 6-[(4'-Aminophenyl)azo]-5-hydroxy-naphthalin-1-sulfonsäure-Natriumsalz (C.I. 14 805), 1,4-Diamino-anthrachinon und 1,4,5,8-Tetraaminoanthrachinon.

7. 3,4,5-Triamino-pyrazol der allgemeinen Formel (I) 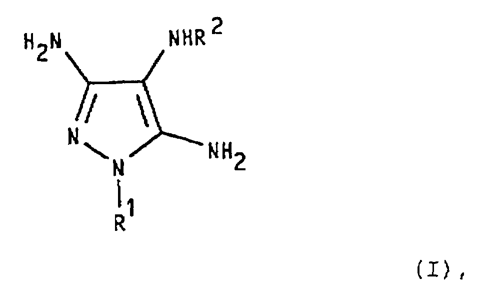 in der R¹ Wasserstoff, Alkyl mit 1 bis 4 Kohlenstoffatomen oder Hydroxyalkyl mit 2 bis 4 Kohlenstoffatomen darstellt und R² Wasserstoff, Alkyl mit 1 bis 4 Kohlenstoffatomen oder Hydroxyalkyl mit 2 bis 4 Kohlenstoffatomen bedeutet.

8. 1-Methyl-3,4,5-triaminopyrazol

9. 3,5-Diamino-1-methyl-4-methylaminopyrazol

10. 3,5-Diamino-4-(2'-hydroxyethyl)amino-1-methylpyrazol

## Claims

1. Agent for the oxidative dyeing of hair, based on a developer substance-coupler substance combination, **characterised in that** it contains as developer substance a 3,4,5-triaminopyrazole of the general formula (I) 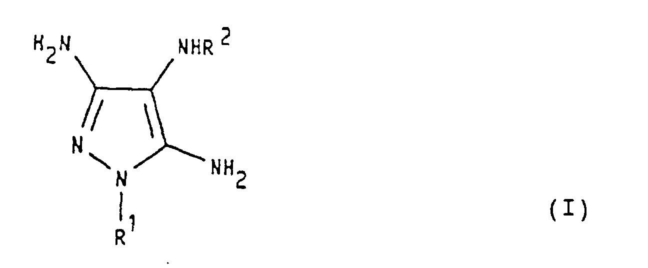 wherein R¹ represents hydrogen, alkyl having from 1 to 4 carbon atoms or hydroxyalkyl having from 2 to 4 carbon atoms and R² represents hydrogen, alkyl having from 1 to 4 carbon atoms or hydroxyalkyl having from 2 to 4 carbon atoms, or the physiologically compatible water-soluble salts thereof.

2. Agent according to Claim 1, **characterised in that** the developer substance of the general formula (I) is selected from 1-methyl-3,4,5-triaminopyrazole, 3,5-diamino-1-methyl-4-methylaminopyrazole and 3,5-diamino-4-(2'-hydroxyethyl)-amino-1-methylpyrazole.

3. Agent according to Claim 1 or 2, **characterised in that** the developer substance of formula (I) is present in an amount of from 0.01 to 3.0% by weight.

4. Agent according to any one of Claims 1 to 3, **characterised in that** the coupler substance is selected from α-naphthol, resorcinol, 4-chlororesorcinol, 2-methylresorcinol, 2-amino-4-(2'-hydroxyethyl)-aminoanisole, 5-amino-2-methylphenol, 3-amino-2-methylphenol, 4-hydroxy-1,2-methylenedioxybenzene, 4-amino-1,2-methylenedioxybenzene, 4-(2'-hydroxyethyl)-amino-1,2-methylenedioxybenzene, 2,4-diaminophenetole, 2,4-diamino-5-methylphenetole, 2,4-diamino-5-fluorotoluene, 4-amino-5-fluoro-2-hydroxytoluene, 2,4-diaminobenzyl alcohol, m-phenylenediamine, 2,4-diaminophenoxyethanol, 4-hydroxyindole, 3-amino-5-hydroxy-2,6-dimethoxypyridine and 3,5-diamino-2,6-dimethoxypyridine.

5. Agent according to any one of Claims 1 to 4, **characterised in that** the total amount of developer substance-coupler substance combination is from 0.1 to 5.0% by weight.

6. Agent according to any one of Claims 1 to 5, **characterised in that** it contains an additional colouring component which is selected from 6-amino-2-methylphenol, 2-amino-5-methylphenol, 4-[(4'-aminophenyl)-(4'-imino-2",5"-cyclohexadien-1"-ylidene)-methyl]-2-methylaminobenzene monohydrochloride (C.I. 42 510), 4-[(4'-amino-3'-methylphenyl)-(4"-imino-3"-methyl-2",5"-cyclohexadien-1"-ylidene)-methyl]-2-methylaminobenzene monohydrochloride (C.I. 42 520), 2-nitro-1,4-diaminobenzene, 2-amino-4-nitrophenol, 2-amino-5-nitrophenol, 2-amino-4,6-dinitrophenol, 2-amino-5-(2'-hydroxyethyl)-aminonitrobenzene, 2-methylamino-5-bis(2'-hydroxyethyl)aminonitrobenzene, the sodium salt of 6-[(4'-aminophenyl)azo]-5-hydroxynaphthalene-1-sulphonic acid (C.I. 14 805), 1,4-diaminoanthraquinone and 1,4,5,8-tetraaminoanthraquinone.

7. 3,4,5-triaminopyrazole of the general formula (I) 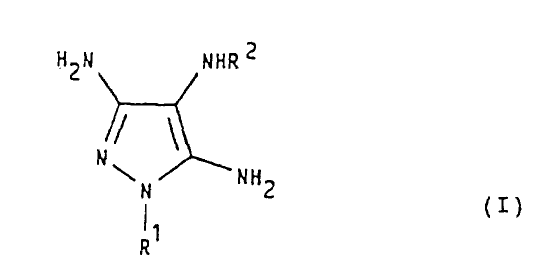 wherein R¹ represents hydrogen, alkyl having from 1 to 4 carbon atoms or hydroxyalkyl having from 2 to 4 carbon atoms and R² represents hydrogen, alkyl having from 1 to 4 carbon atoms or hydroxyalkyl having from 2 to 4 carbon atoms.

8. 1-methyl-3,4,5-triaminopyrazole.

9. 3,5-diamino-1-methyl-4-methylaminopyrazole.

10. 3,5-diamino-4-(2'-hydroxyethyl)-amino-1-methylpyrazole.

## Revendications

1. Agent de coloration oxydative des cheveux, à base d'une combinaison substance de développement-substance de copulation, **caractérisé en ce qu'**il contient à titre de substance de développement un 3,4,5-triaminopyrazol de formule général (I) 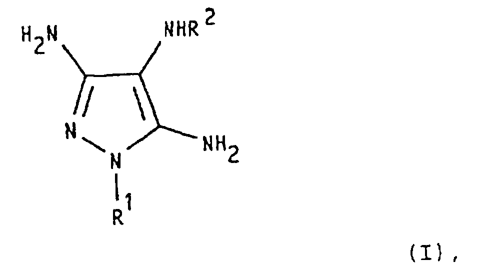 dans laquelle R¹ est l'hydrogène, un radical alkyl ayant de 1 à 4 atomes de carbone ou un radical hydroxyalkyl ayant de 2 à 4 atomes de carbone, et R² est l'hydrogène, un radical alkyl ayant de 1 à 4 atomes de carbone ou un radical hydroxyalkyl ayant de 2 à 4 atomes de carbone, ou leurs sels solubles dans l'eau, physiologiquement compatibles.

2. Agent selon la revendication 1, **caractérisé en ce que** la substance de développement de la formule générale (I) est sélectionnée parmi le 1-méthyl-3,4,5-triaminopyrazol, 3,5-diamino-1-méthyl-4-méthylamino-pyrazol et 3,5-diamino-4-(2'-hydroxyéthyl)amino-1-méthyl-pyrazol.

3. Agent selon la revendication 1 ou 2, **caractérisé en ce que** la substance de développement de formule (I) est contenue en une quantité comprise dans la plage allant de 0,01 à 3,0 pour-cent en poids.

4. Agent selon l'une des revendications 1 à 3, **caractérisé en ce que** la substance de copulation est sélectionnée parmi le α-naphtol, la résorcine, la 4-chlororésorcine, la 2-méthylrésorcine, le 2-amino-4-(2'-hydroxyéthyl)amino-anisol, le 5-amino-2-méthylphénol, le 3-amino-2-méthyl-phénol, le 4-hydroxy-1,2-méthylènedioxybenzène, le 4-amino-1,2-méthylènedioxybenzène, le 4-(2'-hydroxyéthyl)amino-1,2-méthylènedioxybenzène, le 2,4-diamino-phénétol, le 2,4-diamino-5-méthylphénétol, le 2,4-diamino-5-fluoro-toluène, le 4-amino-5-fluoro-2-hydroxytoluène, le 2,4-diamino alcool benzoïque, la m-phénylènediamine, le 2,4-diaminophénoxyéthanol, le 4-hydroxyindol, la 3-amino-5-hydroxy-2,6-diméthoxypyridine et la 3,5-diamino-2,6-diméthoxy-pyridine.

5. Agent selon l'une des revendications 1 à 4, **caractérisé en ce que** la quantité totale de la combinaison substance de développement-substance de copulation est comprise dans la plage allant de 0,1 à 5,0 pour-cent en poids.

6. Agent selon l'une des revendications 1 à 5, **caractérisé en ce qu'**il contient un composant de teinture additionnel, sélectionné parmi le 6-amino-2-méthylphénol, le 2-amino-5-méthylphénol, le 4-[(4'-aminophényl)-(4'-imino-2",5"-cyclohexadiène-1"-ylidène)-méthyl]-2-méthyl-aminoben -zéne-monohydrochlorure (c.l. 42 510), le 4-[(4'-amino-3'-méthyl)phényl)-(4"-imino-3"-méthyl-2",5"-cyclohexadène-1" -ylidène)-méthyl]-2-méthyl-aminobenzène-monohydrochlorure (C.l. 42 520), le 2-nitro-1,4-diaminobenzène, le 2-amino-4-nitro-phénol, le 2-amino-5-nitrophénol, le 2-amino-4,6-dinitrophénol, le 2-amino-5-(2'-hydroxyéthyl)-amino-nitrobenzène, le 2-méthylamino-5-bis-(2'-hydroxyéthyl)-amino-nitrobenzène, le 6-[(4'-aminophényl)azo]-5-hydroxy-naphtaline-1-acide sulfonique-sel de sodium (C.l. 14 805), la 1,4-diamino-anthraquinone et la 1,4,5,8-tétraaminoanthraquinone.

7. 3,4,5-Triamino-pyrazol de formule générale (I) 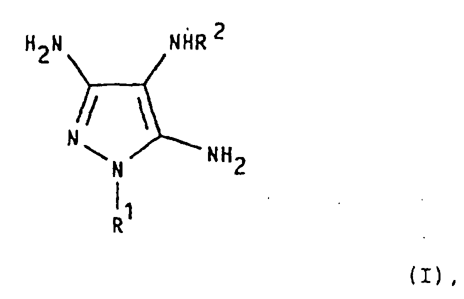 dans laquelle R¹ est l'hydrogène, un radical alkyl ayant de 1 à 4 atomes de carbone ou un radical hydroxyalkyl ayant de 2 à 4 atomes de carbone, et R² est l'hydrogène, un radical alkyl ayant de 1 à 4 atomes de carbone ou un radical hydroxyalkyl ayant de 2 à 4 atomes de carbone.

8. 1-Méthyl-3,4,5-triaminopyrazol.

9. 3,5-diamino-1-méthyl-4-méthylaminopyrazol.

10. 3,5-diamino-4-(2'-hydroxyéthyl)amino-1-méthylpyrazol.
